(19) Europäisches Patentamt
European Patent Office
Office européen des brevets

(11) EP 4 417 190 A1

(12) EUROPEAN PATENT APPLICATION

(43) Date of publication:
21.08.2024 Bulletin 2024/34

(21) Application number: 23382140.4

(22) Date of filing: 15.02.2023

(51) International Patent Classification (IPC):
A61K 8/64 (2006.01)          A61Q 19/08 (2006.01)

(52) Cooperative Patent Classification (CPC):
A61K 8/64; A61Q 19/08

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Lipotrue, S.L.
08850 Gava (ES)

(72) Inventors:
• Grau-Campistany, Ariadna
08028 Barcelona (ES)
• Pastor, Silvia
03540 Alicante (ES)

• Carulla, Patricia
08019 Barcelona (ES)
• Escudero, Juan Carlos
08021 Barcelona (ES)
• Klein, Marco Jan
08902 L Hospitalet de Llobregat (ES)

(74) Representative: Clarke, Modet y Cía., S.L.
C/ Suero de Quiñones 34-36
28002 Madrid (ES)

Remarks:
The complete document including Reference
Table(s) and the Sequence Listing(s) can be
downloaded from the EPO website

(54) CYCLIC PEPTIDES WITH ANTI-AGING EFFICACY

(57) The present invention refers to cycled peptides with anti-aging activity, capable of regulate UPR in endoplasmic reticulum (ER) in response to aging, stress or ROS. Said peptides are capable of affect the expression of genes involving protein misfolding or apoptosis in the ER. Also is disclosed herein, compositions comprising said peptides and their uses in the field of cosmetics, especially against aging signs in the skin.

Fig. 1

**Description**

FIELD OF THE INVENTION

The present invention relates to the field of molecular biology, more precisely to molecular biology applied to cosmetics and medicine, even more precisely to peptides and compositions comprising said peptides with antiaging efficacy.

BACKGROUND ART

In the recent years, there has been a growing interest in the population to prevent, reduce or eliminate skin imperfections and damage caused by endogenous and environmental stressors, in order to maintain the skin with a healthy and youthful appearance. This has led to an increasing interest in the reasons that lead to the appearance of skin imperfections and in the search of compounds and compositions which can prevent or alleviate them.

The skin is the largest organ in humans and due to its location, in the body interface, is subject to intrinsic (chronologic) aging and extrinsic aging, the latter being caused by environmental factors (such as, for example, UV radiation, smoking or pollution). This continuous exposition and, obviously, aging, leads to changes in the skin and to the appearance of imperfections therein (for example, loss of firmness, wrinkling, roughness and/or sagginess).

These factors induce cumulative structural and physiological alterations at the different layers of the skin as well as changes in overall skin appearance (Ganceviciene R et al. (2012). Skin Anti-Aging Strategies. Dermato-Endocrinology, 4, 308-319).

Three primary structural components of the dermis are collagen, elastin and glycosaminoglycans (GAGs), which have been the subjects of the majority of anti-aging research and efforts for aesthetic-anti-aging strategies pertaining to the skin, which range from "anti-wrinkle creams" to various filling agents (Baumann, L (2007). Skin ageing and its treatment. J. Pathol, 211, 241-251.).

In recent years, the number of active ingredients to improve signs of skin aging, such as loss of firmness, skin texture and wrinkling, has increased considerably. Examples of such active ingredients are retinoids, vitamins or botanical extracts (Bradley EJ et al. (2015). Over-the-counter anti-ageing topical agents and their ability to protect and repair photoaged skin. Maturitas 80, 265-272).

On the other hand, linear peptides can also be incorporated in cosmetic formulas to improve the signs of skin aging. Bioactive peptides can imitate body's own molecules and influence processes such as collagen synthesis, with the advantage that they have much better tolerability and stability. In addition, a wide range of activities, chemistries and indications can be developed for them (Zhang L and Falla TJ (2009). Cosmeceuticals and peptides. Clinics in dermatology, 27, 485-494).

Despite the extensive variety of compounds and/or extracts in the field, there is still the need to find new molecules (preferably, peptides) which can be provided for the prevention and/or treatment of aging (preferably, skin aging) and showing a broad spectrum of activities against said aging as well as diseases related to aging. There are certain disadvantages associated with their use. For example, active peptide agents may suffer from poor efficacy of use due to, for instance, their conformational flexibility and/or the easy digestion of peptides by proteases at the sites of intended action. Further, efficacy may be hindered due to the difficulty with which peptides are transported across membranes such as skin and their poor solubility in many cosmetic vehicles.

The human skin is subject to certain ageing processes, some of which are attributable to intrinsic processes (chrono ageing) and some of which are attributable to exogenous factors (environmental, for example photoaging). In addition, temporary or even lasting changes to the skin picture can occur, such as acne, greasy or dry skin, keratoses, rosaceae, light-sensitive, inflammatory, erythematous, allergic or autoimmune-reactive reactions, such as dermatosis and photormatosis. The exogenous factors include, in particular, sunlight or artificial radiation sources having a comparable spectrum, and compounds which can be formed by the radiation, such as undefined reactive photoproducts, which may also be free-radical or ionic. These factors also include cigarette smoke, and the reactive compounds present therein, such as ozone, free radicals, for example the hydroxyl free radical, singlet oxygen and other reactive oxygen or nitrogen compounds which interfere with the natural physiology or morphology of the skin. The influence of these factors can result, inter alia, in direct damage to the DNA of the skin cells and to the collagen, elastin or glycosaminoglycan molecules of the extracellular matrix, which are responsible for the strength of skin. In addition, the signal transduction chains, which are terminated by the activation of matrix-degrading enzymes, may be affected. Important representatives of these enzymes are the matrix metalloproteinases (MMPs, for example collagenases, gelatinases and stromelysins), whose activity is additionally regulated by TIMPs (tissue inhibitors of matrix metalloproteinases). The consequences of the above-mentioned ageing processes are thinning of the skin, weaker interlacing of epidermis and dermis, and a reduction in the number of cells and the supplying blood vessels. These results in the formation of fine lines and wrinkles, the skin becomes leathery, and pigment defects can occur.

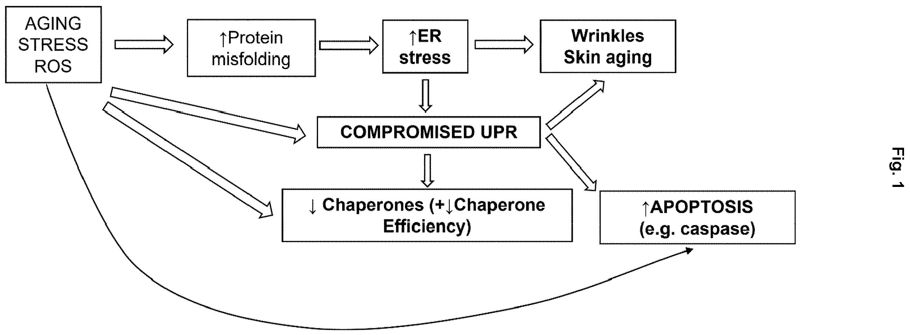

The endoplasmic reticulum (ER) is a multifunctional organelle essential for the synthesis, folding, and processing of secretory and transmembrane proteins. Different factors can disrupt the ER homeostasis resulting in an accumulation of misfolded and unfolded proteins, a condition known as "ER stress activating the Unfolded Protein Response (UPR)". The UPR transduces information about the protein-folding status in the ER lumen to the nucleus and cytosol to buffer fluctuations in unfolded protein load. When cells undergo an irreversible ER stress that overcomes UPR capacity, this pathway eliminates damaged cells by apoptosis (Hetz C (2012). The unfolded protein response: controlling cell fate decisions under ER stress and beyond. Nat Rev Mol Cell Biol. 13, 89-102). It has been described that the ability to activate the UPR declines with age, while its constitutive activation can extend longevity (Taylor RC (2016). Aging and the UPR(ER). Brain Res. 1648: 588-93). Factors such as aging, stress or radical oxygen species (ROS) leads to an increase of the protein misfolding, for example, carbonylation of proteins or glycation and lipoxidation which lead to Advanced Glycation End-Products (AGEs) and Advanced lipoxidation end-products (ALEs) all of which produce an increase of the ER stress. Moreover, aged cells have shown to have a decreased number of chaperones and moreover, the limited chaperones that are still present in the aging ER appear to be impaired. Additionally, aging also appears to alter the threshold at which the UPR switches to the apoptotic pathway. Taken all together, aging causes a decrease of chaperones (or chaperone efficiency), increases apoptosis and eventually, causes skin aging and the appearance of wrinkles, since the UPR activation cannot rescue this ER stress. **Figure 1** shows a scheme of the effect of aging in the ER and effect in the UPR.

As already mentioned, during ageing, the function of the UPR is compromised as there is a progressive failure of the chaperoning systems and a decline in UPR components. The ER stress leads to the loss of calcium homeostasis and accumulation of unfolded protein, which causes cell dysfunction and cell death.

Calreticulin (CRT or CALR) is a calcium-binding protein which chaperones glycoproteins through the endoplasmic reticulum (ER), ensuring proper protein folding and preventing aggregation. A major function of this protein is to maintain intracellular calcium homeostasis, important in signaling, particularly of integrins. CALR represents one of the most critical calcium storage proteins because over 50 % of the entire calcium in the cell is bound to this chaperone. Down-regulation of CALR results in mitochondrial calcium overload and induction of mitochondria permeability transition pore (mPTP)-dependent cell death. In addition, CALR regulates cellular responses to stress and in concert with other ER chaperone proteins, identifies misfolded proteins for proteasome-mediated destruction outside the ER. In addition, it plays a key role as inducer of unfolded protein response (UPR) (Michalak M et al. (2009) Calreticulin, a multi-process calcium-buffering chaperone of the endoplasmic reticulum. Biochem J, 417: 651-33). Moreover, CALR exists in the cytoplasm, cell surface, extracellular space and plasma; being a multicompartmental protein that regulates a wide array of intracellular and extracellular responses, including extracellular matrix ECM production. It has been observed that CALR stimulates TGFβ-3 synthesis, which induces the production of different ECM components, such as integrins or fibronectin, which can counteract the effect of aging on the ECM (Owusu BY et al. (2018). The role of the endoplasmic reticulum protein calreticulin in mediating TGF-β-stimulated extracellular matrix production in fibrotic disease. J Cell Commun Signal. 12, 289-99); Pandya UM et al. (2020) Calreticulin exploits TGF-β for extracellular matrix induction engineering a tissue regenerative process. FASEB J. 34: 15849-74).

Binding immunoglobulin protein (BiP) is an Hsp70 ATP-dependent chaperone that plays a critical role in UPR, it is actually considered the master regulator of ER function. BiP is a chaperone located in the lumen of the ER and its main function is to enforce protein folding. Under ER stress, unfolded proteins in the lumen of the ER dramatically increase. After nascent chains enter the lumen, they are directly bound to BiP and the folding process is started. The expression of BiP is highly upregulated upon ER stress by all three arms of the UPR. A high level of BiP in the ER lumen decreases the level of unfolded protein and could lead to a stop of the UPR (Taouji S et al. (2013). Chapter Seventeen - Oligomerization in Endoplasmic Reticulum Stress Signaling. Progress in Molecular Biology and Translational Science. 117, 2013, Pages 465-84). BiP is a direct ER stress sensor, leading to UPR activation through its interaction with proteins IRE1 and PERK (Kopp *et al.,* 2019).

It is therefore an object of the instant disclosure to provide advantageous peptides and compositions comprising therefrom for cosmetic applications which provide enhanced efficacy, to provide advantageous peptides for cosmetic applications which inter alia provide higher resistance to proteolytic degradation and/or to provide advantageous peptides for cosmetic anti-ageing applications, activating the UPR and increasing the ER chaperones. These peptides would contribute to the correct protein folding of proteins, leading to a youthful appearance and smooth skin, decreasing apoptosis and ER stress.

BRIEF DESCRIPTION OF INVENTION

The present invention is defined in the appended claims.

In one first aspect, the invention is directed to a cycled peptide of between 5 and 10 amino acids capable of increase ER chaperones expression; its acceptable isomers, salts, solvates, derivatives and/or mixtures thereof.

In a second aspect, the invention is directed to a cosmetic composition comprising a cosmetically effective amount of the cycled peptide as defined in the first aspect.

In a third aspect, the invention is directed to the use of the peptide or the cosmetic composition to reduce, prevent and/or eliminate signs of skin aging.

In a fourth aspect, the invention is directed to a pharmaceutical composition comprising the cycled peptide.

In a fifth aspect, the invention is directed to the peptide or the pharmaceutical composition for use as a medicament.

In a sixth aspect, the invention is directed to the peptide or the pharmaceutical composition for use in a method to reduce, prevent and/or eliminate signs of skin aging in a subject.

BRIEF DESCRIPTION OF DRAWINGS

Figure 1. Scheme of the effect of the aging, stress and ROS on ER.

Figure 2. (A) Modulation of aging-related genes TGFBR3, FN, ITGB1, ITGA5 and CALR in HEKa cells treated with cycled peptide B for 24 hours at 0.01 mg/mL. (B) Modulation of aging-related genes TGFBR3, ITGB1 and ITGA5 in HDFa treated 6 h with cycled peptide B at 0.05 mg/mL.

**Figure 3.** Fluorescence quantification of CALR on Human Epidermal Keratinocytes after 24 hours of treatment with 0.005 mg/mL of cycled peptide B. The black column represents the untreated cells, and the grey column represents the cells treated with the cycled peptide. Graphic shows the mean $\pm$ standard error of the mean (SEM).

**Figure 4.** BiP levels on Human Epidermal Keratinocytes after 24 hours of treatment with cycled peptide B (grey columns) compared with untreated cells (black column). Graphic shows the mean $\pm$ standard error of the mean (SEM).

**Figure 5. (A)** Caspase 3 activity in HEKa cells treated with cycled peptide A and BFA (%) (1: basal; 2: BFA 0.05X; 3: peptide A 0.0025 mg/mL + BFA 0.05X; 4: peptide A 0.01 mg/mL + BFA 0.05X; 6: peptide A 0.05 mg/mL + BFA 0.05X). **(B)** Caspase 3 formation in HEKa cells treated with linear peptide B and BFA (%) (1: basal; 2: BFA 0.05X; 3: peptide B linear 0.005 mg/mL + BFA 0.05X; 4: peptide B linear 0.01 mg/mL + BFA 0.05X; peptide B cycled 0.005 mg/mL + BFA 0.05X).

**Figure 6. (A)** Expression of collagen genes COL3A1, COL5A1, COL6A1 and COL7A1 after 6 hours of treatment with 0.001 mg/mL of peptide B (grey) and its linear form (black) in HDFa cells. (B) Expression of collagen genes COL1A1 and COL4A1 after 6 hours of treatment with 0.1 mg/mL of peptide B (grey) and its linear form (black). **(C)** Expression of metalloproteases MMP1 and MMP3 after 24 hours of treatment with 0.01 mg/mL of peptide B (grey) and its linear form (black) in HDFa cells.

**Figure 7.** TGFB3 expression in HDFa cells treated with conditioned media (KCM) from cycled peptide B treated keratinocytes (Black: KCM, grey: KCM treated with 0.0025 mg/mL or 0.01 mg/mL of peptide B).

**Figure 8.** COLI relative expression in HDFa cells treated with TGFB1 or conditioned media (KCM) from cycled peptide B treated keratinocytes. Basal KCM, 10 ng/mL TGFb1, 0.005 mg/mL and 0.01 mg/mL of cycled peptide B.

DETAILED DESCRIPTION OF THE INVENTION

The inventors of the present invention have surprisingly found that cycled peptides provide for the appropriate cosmetic anti-wrinkling activity. Said peptides are capable of inducing CALR expression and decrease apoptosis in cells. Moreover, the peptides of the present invention are also able to upregulate the expression of key extracellular matrix (hereinafter, ECM) components, at the gene and protein expression levels, such as collagen I and TGFB3. Altogether validates the usefulness of the peptides of the present invention in cosmetics for the treatment, prevention and/or reduction aging in cells, such as wrinkles; treatment, prevention and/or reduction of sagging or looser skin; treatment, prevention and/or reduction of skin roughness; and improvement of skin firmness and/or elasticity.

In addition, the peptides of the present invention, due to their structure have inherent advantages, as, for example, increased or prolonged stability and increased or improved penetration, preferably, through the skin.

The present invention refers to cyclic peptides and their use in cosmetic and pharmaceutical compositions. By means of cyclizing specific linear peptides, it has been obtained totally novel molecules with a completely new mechanism of action aiming to target the ER response.

In some embodiments, the peptide of the present invention is capable of stimulate UPR in ER. The peptides of the present invention perform this activity by upregulating the expression of relevant genes and proteins from the ECM; more preferably, the peptide of the present invention is also capable of upregulating at least one gene and/or protein in charge of the synthesis and/or of the maintenance of the ECM; more preferably, the peptide of the present invention is also capable of upregulating at least one gene and/or protein in charge of the synthesis and/or of the maintenance of the ECM.

In a first aspect, the present invention refers to a cycled peptide capable of modulate the expression of ER chaperones. Preferably, the cycled peptide modulates the expression of a least a chaperone selected from CALR and/or BiP. More preferably, the at least one chaperone is CALR.

Cyclization is known to be an effective strategy to enhance stability against enzymatic and chemical degradation. Cyclized (or cycled) peptides tend to have better permeability and stability because they are less prone to hydrolysis of exo- and endopeptidases. The elimination on the charged termini may enhances membrane permeability. These peptides may be used to synthesize analogues that mimic the biological activity of natural structures enhancing its bioavailability, reducing conformational flexibility that often lead to a better interaction with its target. In summary, cycled peptides may produce an improved biological activity over their linear forms. However, there is not information available of cycled peptides in compositions with an improved activity over their linear counterparts.

The cyclization of peptides may be produced by:

- Head-to-tail (end-to-end, C-terminus to N-terminus) cyclization (amide bond formation between amino and carboxyl termini, many biologically active cyclic peptides are formed this way).
- End-to-side chain cyclization (requires a trifunctional amino acid, either head-to-side chain or side chain-to-tail).
- Side chain-to-side chain cyclization (requires 2 trifunctional amino acids).

(White C and Yudin A (2011). Contemporary strategies for peptide macrocyclization. Nature Chem 3, 509-524).

In a preferred embodiment, the present invention refers to a cycled peptide of between 5 to 10 amino acids, its acceptable isomers, salts, solvates derivatives and/or mixtures thereof, capable to stimulate ER chaperone expression, preferably CALR. More preferably, the peptide is an hexapeptide, an heptapeptide, or an octapeptide. Too short or too long peptides are more complicated to cycle (Claro B et al. (2018). Design and applications of cyclic peptides. Peptide Applications in Biomedicine, Biotechnology and Bioengineering, 87-129).

It is contemplated that the amino acids used or present in the peptides of the present invention are L-amino acids, D-amino acids or combinations thereof. In a preferred embodiment, the amino acids used or present in the peptides of the present invention are L-amino acids.

Preferably, the isomers mentioned above are stereoisomers. It is contemplated that said stereoisomers are enantiomers or diastereoisomers. Hence, in a preferred embodiment of the present invention, the peptide is a racemic mixture, a diastereomeric mixture, a pure enantiomer or a pure diastereoisomer.

In addition, isomers, salts, solvates, derivatives and/or mixtures thereof are, preferably, cosmetically acceptable isomers, salts, solvates, derivatives and/or mixtures thereof.

In the preferred embodiment of the first aspect of the invention, the cyclic peptide is an hexapeptide as represented below:

AA1 -AA2-AA3-AA4-AA5-AA6

wherein

AA1 is selected from an aromatic amino acid;
AA2 is selected from an aromatic amino acid;
AA3 is selected from a positively charged amino acid;
AA4 is Ala;
AA5 is Leu; and
AA6 is selected from a positively charged amino acid.

The cycled peptides of the invention are cycled by head-to-tail. The peptide is synthesized without acetylation and the cycle is closed by an amide bond.

In a more preferred embodiment of the first aspect of the invention, the cyclic peptide is an hexapeptide as represented below:

AA1 -AA2-AA3-AA4-AA5-AA6

wherein

AA1 is selected from Trp or Phe;

AA2 is selected from Trp or Phe;

AA3 is selected from His or Arg;

AA4 is Ala;

AA5 is Leu; and

AA6 is selected from His or Arg.

In an even more preferred embodiment, the peptide is selected from the group consisting of:

cyclo-Trp-Phe-Arg-Ala-Leu-His (peptide A, SEQ ID NO:1) and

cyclo-Phe-Trp- His- Ala-Leu-Arg (peptide B, SEQ ID NO:2).

The term "cyclo" means that the sequence may cycle from any of the amino acids of the sequence.

In the most preferred embodiment, the peptide is:

cyclo- Phe-Trp- His- Ala-Leu-Arg (peptide B, SEQ ID NO:2).

Within the scope of the present invention are also included peptides derived or obtained from any of the peptides disclosed above, preferably incorporating one or more conservative substitutions, as long as they retain or show or improve at least one of the activities mentioned above (and, preferably, all of the activities mentioned above for the peptides of the present invention).

In addition, also included within the scope of the present invention are peptides with a 70% percentage identity, preferably 80%, more preferably 90%, more preferably 95%, even more preferably 99% percentage identity with any of the specific sequences mentioned above as long as they retain or show or improve at least one of the activities mentioned above (and, preferably, all of the activities mentioned above for the peptides of the present invention).

The peptides of the invention provide new cosmetic ingredients of improved anti-aging effect of their linear counterparts. The inventors have characterized the biological activity of these molecules and the mechanism of action of these peptides have been substantiated by means of qPCR, ELISA and Immunofluorescence (IF).

The peptides of the present invention may be synthesized and produced by any means known in the state of the art. For example, they may be synthesized and produced by chemical synthesis (preferably, by means of solid phase peptide synthesis), expressing said peptides in cell cultures or by means of transgenic production of the peptide in plants or animals. In addition, the peptides of the present invention may be purified by any means known in the state of the art.

In a second aspect, the present invention refers to a cosmetic composition comprising a cosmetically effective amount of the peptide of the present invention. Hence, the cosmetically effective amount of the at least one peptide of the present invention comprises from 0.0001 % (weight/volume in g/100mL, hereinafter, w/v) to 0.05 % (w/v) of a peptide of the present invention. In a most preferred embodiment, the cosmetic composition of the present invention comprises from 0.0001 % (w/v) to 0.005 % (w/v) of a peptide of the present invention, more preferably, from 0.00025 % (w/v) y 0.00075 % (w/v).

It is contemplated that the cosmetic composition of this second aspect of the invention comprises at least one additional cosmetic ingredient. Said additional cosmetic ingredient can be at least one excipient and/or at least one additional cosmetic active ingredient. The additional cosmetic ingredient can be any cosmetic ingredient known in the state of the art as long as it does not affect, or it does not affect in an unacceptable manner the activity of the peptides of the present invention. The additional cosmetic ingredients comprise those usually used in the state of the art as, for example, adjuvants such as stabilizers, solubilizers, vitamins, colorants and perfumery; carriers; and/or other cosmetic ingredients.

Said additional cosmetic ingredients may be of a synthetic or natural origin, such as, for example, plant extracts, or they can be derived from a biofermentation process (see, for example, CTFA Cosmetic Ingredient Handbook, Eleventh Edition (2006)).

It is contemplated that the additional cosmetic ingredients mentioned above comprise those ingredients commonly used in compositions for caring for; cleaning skin and/or deodorants and/or creams to prevent hyperhidrosis; such as, for example, agents inhibiting melanin synthesis, whitening or depigmenting agents, anti-aging agents, agents inhibiting NO-synthase, antioxidants, anti-atmospheric pollution and/or free radical trapping agents, anti-glycation agents, emulsifying agents, emollients, organic solvents, liquid propellants, skin conditioners such as for example wetting agents, moisture retaining substances, alpha hydroxy acids, moisturizers, vitamins, pigments or colorants, dyes, gelling polymers, thickeners, surfactants, softeners, other anti-wrinkle agents, agents capable of reducing or eliminating bags under the eyes, exfoliating agents, antimicrobial agents, antifungal agents, bactericides, agents stimulating dermal or epidermal macromolecule synthesis and/or capable of preventing or inhibiting their degradation, such as for example agents stimulating collagens synthesis, agents stimulating elastin synthesis, agents stimulating laminin synthesis, agents inhibiting collagen degradation, agents inhibiting elastin degradation, agents stimulating fibroblast proliferation, agents stimulating keratinocyte proliferation, agents stimulating keratinocyte differentiation, agents stimulating lipid synthesis and synthesis of components of the *stratum corneum* (ceramides, fatty acids, etc.), dermorelaxing agents, agents stimulating glycosaminoglycan synthesis, DNA repairing agents, DNA protecting agents, agents stimulating proteosome activity, anti-pruritus agents, agents for treating sensitive skin, reaffirming agents, astringent agents, sebum production regulating agents, agents stimulating lipolysis, anti-cellulite agents, calming agents, anti-inflammatory agents, agents acting on capillary circulation and/or microcirculation, agents acting on cell mitochondria, agents intended to improve the dermo-epidermal junction, preservatives, perfumes, chelating agents, plant extracts, essential oils, marine extracts, agents derived from a biofermentation process, mineral salts, cell extracts and/or solar filters (organic or mineral photoprotective agents active against ultraviolet A and B rays) among others.

In an embodiment, at least one of the additional cosmetic ingredients is a cosmetic active principle or substance which may exert the same, similar, complementary or different cosmetic activities as those disclosed above for the peptides of the present invention. It is contemplated that the cosmetic composition of the present invention comprises other anti-wrinkling or antiaging agents, for example, collagen, elastin, growth factors, hyaluronic acid boosters, barrier function agents, illuminating agents, agents stimulating the expression and/or synthesis of collagen I, III, IV and/or VI and laminin; agents stimulating the synthesis of glycosaminoglycans or hyaluronic acid; agents stimulating the expression and/or synthesis of elastin and other elastic fibers-related proteins; agents inhibiting collagen and/or elastic fibers degradation; agents stimulating the expression and/or synthesis of mitochondria-related proteins (for example, sirtuins and aconitase); agents stimulating the expression and/or synthesis of focal adhesion proteins; agents stimulating keratinocytes and/or fibroblasts proliferation and/or differentiation; antioxidants; anti-atmospheric pollution and/or free radical trapping agents; anti-glycation agents; detoxifying agents; agents decreasing chronological aging, environmental aging and inflammation aging; and agents decreasing melanin production and/or inhibiting tyrosinase and/or agents stimulating lipid synthesis and synthesis of components of the epidermis (keratins) and more specifically the stratum corneum (keratins, ceramides, filaggrin, loricrin and SPRR1B).

In addition, the cosmetic composition of the present invention can be formulated in any form usually used in the state of the art as, for example, solution, suspension, emulsion, paste, gel, cream, powder, spray, lotion, oil, liniment, serum, mousse, ointment, bar or pencil including "leave on" and "rinse-off" formulations. The cosmetic composition of the present invention can also be incorporated by means of techniques known in the state of the art to different types of solid accessories such as towelettes, hydrogels, adhesive (or non-adhesive) patches or face masks, or it could be incorporated to different make-up line products such as concealers, make-up foundations, lotions or make-up removal lotions, among others.

It is also contemplated that the cosmetic composition of the present invention can also be incorporated in cosmetic sustained release systems and/or carriers such as liposomes, milliparticles, microparticles and nanoparticles, as well as in sponges, vesicles, micelles, millispheres, microspheres, nanospheres, lipospheres, millicapsules, microcapsules and nanocapsules, as well as in microemulsions and nanoemulsions, for the purpose of obtaining greater penetration of the active ingredient.

In a preferred embodiment, the cosmetic composition of the present invention is suited or adapted to be applied by means of iontophoresis, more preferably, in the face and/or the body of a subject, more preferably, in the face, neck, hands and/or armpits of a subject, even more preferably in the face and/or neck of a subject (preferably, a human).

In a further preferred embodiment, which can be combined with any of the embodiments disclosed above, the peptide of the present invention is suited or adapted to be applied subcutaneously, more preferably, in the face, neck, of a subject of a subject (preferably, a human).

In the most preferred embodiment, the cosmetic composition of the present invention is suited or adapted to be applied topically (more preferably, in the form of a cream), more preferably, in the face and/or the body of a subject, more preferably, in the face, neck, of a subject, even more preferably in the face and/or neck of a subject (preferably, a human).

In an embodiment, the cosmetic composition of the present invention consists essentially of peptides of the present invention.

In another embodiment, the cosmetic composition of the present invention consists of peptides of the present invention.

As it is directly derivable from the examples included below, the peptides of the present invention have the required activities to provide for the prevention, treatment and/or reduction of aging effect by stimulation of the UPR of the ER by increasing ER chaperones such as CALR or BiP. In addition, the peptides of the present invention also have the required activities to provide for the prevention, treatment and/or reduction of looser or sagging skin and skin roughness (preferably, wrinkle roughness); as well as for the improvement of skin firmness and elasticity. Therefore, the peptides of the present invention solve the technical problem mentioned above and are useful in cosmetics.

In a third aspect, the invention refers to the cosmetic use of the peptide of the present invention according to the first aspect of the invention, or of the cosmetic composition as defined in the second aspect of the invention, to reduce, prevent and/or eliminate signs of skin aging.

The peptide or the composition are used in a cosmetically effective amount or quantity.

In a preferred embodiment, the use as a cosmetic is to reduce, prevent and/or eliminate signs of skin aging or the improvement of skin firmness and/or elasticity. In a more preferred embodiment, the use is to prevent and/or eliminate signs of skin aging.

Preferably, the signs of skin aging are wrinkles, skin roughness (preferably, wrinkle roughness), dullness, pigmentation, looser skin or sagging skin, more preferably, wrinkles, skin roughness, non-inflammatory looser skin or non-inflammatory sagging skin, more preferably, wrinkles, even more preferably facial wrinkles. Said wrinkles can be chronological wrinkles or expression wrinkles.

Non-inflammatory looser skin is equivalent to cosmetic looser skin, this is, non-pathological (non-medical condition).

Non-inflammatory sagging skin is equivalent to cosmetic sagging skin, this is, non-pathological (non-medical condition).

As it is directly derivable from what it has been stated above, in the present invention, signs of skin aging (preferably, wrinkles, both chronological and expression wrinkles) are prevented, reduced and/or eliminated (by the peptide of the present invention or by the composition of the present invention) by means of stimulating ER chaperones.

In this third aspect of the present invention, the subject in need of the cosmetic treatment is preferably a mammal, more preferably, a human.

In a fourth aspect, the invention refers to a pharmaceutical composition comprising the cycled peptide of the invention.

It is contemplated that the pharmaceutical composition comprises one type of peptide of the present invention or a combination or mixture of different peptides of the present invention.

The pharmaceutical composition of the present invention comprises a pharmaceutically effective amount of the at least one peptide of the present invention.

The pharmaceutical composition can be in any form known in the state of the art which is suited for the chosen route. It is contemplated that the pharmaceutical composition of the present invention is suited or adapted to be administered by any means and any route known in the state of the art (for example, subcutaneously, intramuscularly, intravenously, topically or orally.

It is contemplated that the pharmaceutical composition of the present invention also comprises at least one additional pharmaceutical ingredient. Said additional pharmaceutical ingredient can be at least one excipient and/or at least one additional pharmaceutical active ingredient.

In a fifth aspect, the present invention refers to a cosmetic method to reduce, prevent and/or eliminate signs of skin aging in a subject comprising the administration of a cycled peptide of the present invention or the cosmetic composition as described above to a subject, preferably a human.

In a sixth aspect, the present invention refers to the cycled peptide as disclosed above or the pharmaceutical composition as described in the fourth aspect of the invention, for use as a medicament.

In a seventh aspect, the present invention refers to the cycled peptide as disclosed above or the pharmaceutical composition for use in a method to reduce, prevent and/or eliminate signs of skin aging in a subject.

As used herein, the term "cosmetic composition" or preferably more briefly just "composition" in accordance with the present instant disclosure preferably relates to a formulation that can be used for cosmetic purposes, purposes of hygiene and/or as a basis for delivery of one or more pharmaceutical ingredients. It is also possible that these formulations are used for two or more of these purposes at one time. Thus, the terms "cosmetics," "cosmetic composition" and/or "composition" as used herein, preferably include any product applicable to the skin without limitation

The "pharmaceutical composition" in accordance with the present instant disclosure preferably includes, without limitation, carriers for dermatological purposes, including topical and transdermal application of pharmaceutically active ingredients. These can be in the form of gels, patches, creams, lotions, emulsions, colloids, solutions, suspensions, powders and the like. Compositions in accordance with the instant disclosure preferably include cosmetics, personal care products and pharmaceutical preparations.

As used herein, the terms "skin ageing" or "signs of skin ageing" preferably include, but are not limited to, all outward visibly and tactilely perceptible manifestations as well as any other macro or micro effects due to skin ageing. Such signs may be induced or caused by intrinsic factors or extrinsic factors, e.g., chronological ageing and/or environmental damage. The signs may result from processes which preferably include, but are not limited to, the development of textural discontinuities, such as wrinkles and coarse deep wrinkles, skin lines, crevices, bumps, large pores (e.g. associated with adnexal structures such as sweat gland ducts, sebaceous glands, or hair follicles), or unevenness or roughness of the skin, loss of skin elasticity (loss and/or in activation of functional skin elastin), sagging (including puffiness in the eye area and jowls), loss of skin firmness, loss of skin tightness, loss of skin recoil from deformation, discoloration (including (black) under eye circles), blotching, sallowness, sallowness, hyperpigmented skin regions such as age spots and freckles, keratoses, abnormal differentiation, hyperkeratinization, elastosis, collagen breakdown, and other histological changes in the stratum corneum, dermis, epidermis, the skin vascular system (e.g., telangiectasia or spider vessels), and underlying tissues, especially those proximate to the skin. Particularly preferred in accordance with the present instant disclosure, the signs of skin aging are wrinkles and the compositions of the present instant disclosure are, in certain preferred embodiments, useful in fighting, treating or preventing wrinkles.

As used herein, the term "derivative" and its plural, refer to cosmetically acceptable compounds, this is, derived from the compound of interest that can be used in the preparation of a cosmetic, and to cosmetically unacceptable derivatives since these may be useful in the preparation of cosmetically acceptable derivatives.

As used in the present document, the term "salt" and its plurals refer to any type of salt from among those known in the state of the art, for example, halide salts, hydroxy acid salts (such as oxyacid salts, acid salts, basic salts and double salts), hydroxy salts, mixed salts, oxy salts or other hydrated salts. This term comprises cosmetically acceptable salts; and cosmetically unacceptable salts since the latter may be useful in the preparation of cosmetically acceptable salts.

As used in the present document, the term "isomer" and its plural refer to optical isomers, enantiomers, stereoisomers or diastereoisomers. The individual enantiomers or diastereoisomers, as well as their mixtures, may be separated by conventional techniques known in the state of the art.

As used herein, the term "solvate" and its plural refer to any solvate known in the state of the art, such as polar, apolar or amphiphilic solvates, and include any cosmetically acceptable solvate which, when administered or applied to the interested subject (directly or indirectly) provides the compound of interest (the peptide or peptides of the present invention). Preferably, the solvate is a hydrate, a solvate with an alcohol such as methanol, ethanol, propanol or isopropanol, a solvate with an ester such as ethyl acetate, a solvate with an ether such as methyl ether, ethyl ether or THF (tetrahydrofuran) or a solvate with DMF (dimethylformamide), and more preferably a hydrate or a solvate with an alcohol such as ethanol.

In addition, as used herein, the term "amino acid" and its plural include the amino acids codified by the genetic code as well as uncodified amino acids, whether they are natural or not and whether they are D- and L-amino acids. Examples of uncodified amino acids are, without restriction, citrulline, ornithine, sarcosine, desmosine, norvaline, 4-aminobutyric acid, 2-aminobutyric acid, 2-aminoisobutyric acid, 6-aminohexanoic acid, 1-naphthylalanine, 2-naphthylalanine, 2-aminobenzoic acid, 4 aminobenzoic acid, 4-chlorophenylalanine, 2,3-diaminopropionic acid, 2,4 diaminobutyric acid, cycloserine, carnitine, cysteine, penicillamine, pyroglutamic acid, thienylalanine, hydroxyproline, allo-isoleucine, allo-threonine, isonipecotic acid, isoserine, phenylglycine, statin, β-alanine, norleucine, N-methylamino acids, α-amino acids and β-amino acids, among others, as well as their derivatives. Nevertheless, further unnatural amino acids are known in the state of the art (see, for example, "Unusual amino acids in peptide synthesis" by D. C. Roberts and F. Vellaccio, The Peptides, Vol. 5 (1983), Chapter VI, Gross E. and Meienhofer J., Eds., Academic Press, New York, USA).

The "percentage of identity" regarding peptides, polypeptides and proteins, as used herein, has the meaning commonly attributed in the state of the art and, hence, relates to the percentage of amino acids which are identical between two amino acid sequences which are compared after an optimal alignment of these sequences, where said percentage is merely statistical and the differences between the two amino acid sequences are randomly distributed throughout the sequence. "Optimal alignment" is understood as that alignment of amino acid sequences giving rise to a greater percentage of identity. The percentage of identity is calculated by determining the number of identical positions in which an amino acid is identical in the two compared sequences, dividing the number of identical positions by the number of compared positions and multiplying the result obtained by 100 to obtain the percentage of identity between the two sequences. The sequence comparisons between two amino acid sequences can be carried out manually or by means of computer programs known in the state of the art, such as the BLAST (Basic Local Alignment Search Tool) algorithm.

The antiaging effect of the cycled peptides is proven by the upregulation of anti-aging described genes. Specially, the cycled peptides increase the expression of chaperones such as CALR and BiP, which suggest the role of the peptides as enhancer of proper protein folding and UPR. The results shown herein strongly suggest the role of these peptides to improve the cellular response to endoplasmic reticulum stress, with applications for antiaging products development. In addition, it has been found that the peptides of the invention protect the cells from apoptosis caused by ER stress.

SEQUENCE LISTING FREE TEXT

SEQ ID NO 1: WFRALH
SEQ ID NO: 2 FWHALR

EXAMPLES

**Abbreviations:** The abbreviations used in the present text for amino acids follow the 1983 IUPAC-IUB Joint Commission on Biochemical Nomenclature recommendations outlined in Eur. J. Biochem. (1984) 138:937.

**For the examples:** Ala (alanine); Arg (arginine); BOP (Benzotriazole-1-yl-oxy-tris-(dimethylamino)-phosphonium); BSA (Bovine Serum Albumin); cDNA (Complementary DNA); CTC (2-Chlorotrityl chloride resin); C-terminal (carboxy-terminal); DAPI (4',6-diamidino-2-phenylindole); DCM (dichloromethane); DIPCDI (N,N'-diisopropylcarbodiimide); DMF (N,N-dimethylformamide); DMSO (Dimethyl sulphoxide); DNA (Deoxyribonucleic acid); equiv (equivalent); ESI-MS (electrospray ionization mass spectrometry); Fmoc (9-fluorenylmethyloxycarbonyl); His (histidine); HOBt (1-hydroxybenzotriazole); HPLC (high performance liquid chromatography); Leu (Leucine); LSGS (Low Serum Growth Supplement); Me (methyl); MeCN (acetonitrile); MeOH (methanol); MTT ((3-[4,5-dimethylthiazol-2-yl]-2,5-diphenyltetrazolium bromide); N-terminal (amino-terminal); PBS (Phosphate Buffered Saline); Phe (phenylalanine); qPCR (real-time polymerase chain reaction); RH (Relative Humidity); RIPA buffer (Radioimmunoprecipitation assay buffer); RNA (Ribonucleic acid); RT (room temperature); RT-qPCR (Quantitative reverse transcription Polymerase Chain Reaction); tBu (tert-butyl); TFA (trifluoroacetic acid); TIS (triisopropylsilane); Trt (triphenylmethyl or trityl); Trp (tryptophan).

**Chemical synthesis procedures included in the examples:** it is noted that all synthetic processes were carried out in polypropylene syringes fitted with porous polyethylene discs or Pyrex® reactors fitted with porous plates. All the reagents and solvents were synthesis quality and were used without any additional treatment. The solvents and soluble reagents were removed by suction. The Fmoc group was removed with piperidine-DMF (2:8, volume/volume, v/v) (at least $1\times1$ min, $2\times10$ min, 5 mL/g resin) (Lloyd Williams P. et al., Chemical Approaches to the Synthesis of Peptides and Proteins, CRC, 1997, Boca Raton (Fla., USA)). Washes between stages of deprotection, coupling, and, again, deprotection, were carried out with DMF ($3\times1$ min) and DCM ($3\times1$ min) each time using 10 mL solvent/g resin. Coupling reactions were performed with 3 mL solvent/g resin. The control of the couplings was performed by carrying out the ninhydrin test (Kaiser E. et al., Anal. Biochem, 1970, 34: 595598). All synthetic reactions and washes were carried out at RT.

### *Example 1. Synthesis and preparation of the peptides*

1.1 <u>Obtaining Fmoc-AA1-AA2-AA3-AA4-AA5-AA6-CTC-resin</u>

(wherein AA1 is L-Leu; AA2 is L-His; AA3 is L-Trp; AA4 is L-Phe; AA5 is L-Arg and AA6 is L-Ala)

Weights were normalized. In 4.8 g (2.5 mmol) of the CTC resin with a functionalization of 0.52 mmol/g, 2.33 g of Fmoc-L-Ala-OH (7.5 mmol; 3 equiv) were incorporated onto the resin in the presence of DIEA (3 equiv) using DCM/DMF as a solvent for two hours.

The resin was then washed as described in the general methods known in the state of the art and the deprotection treatment of the Fmoc group was performed using piperidine-DMF to couple the next amino acid. Following the previously described protocols 4.86 g of Fmoc-L-Arg(Pbf)-OH (7.5 mmol; 3 equiv); subsequently 2.90 g of Fmoc-L-Phe-OH (7.5 mmol; 3 equiv); subsequently 3.94 g of Fmoc-L-Trp(Boc)-OH (7.5 mmol; 3 equiv); subsequently 4.64 g Fmoc-L-His(Trt)-OH (7.5 mmol; 3 equiv), and subsequently 2.65 g Fmoc-L-Leu-OH (7.5 mmol; 3 equiv) were coupled, sequentially, each coupling in the presence of 1.01 g of HOBt (7.5 mmol; 3 equiv) and 1.17 mL of DIPCDI (7.5 mmol; 3 equiv). As already noted above, between each amino acid addition step, a deprotection treatment of the Fmoc group was performed.

After the synthesis, the peptide resins were washed with DCM (5 times for 3 minutes each one) and dried under vacuum.

Using the synthesis procedures mentioned above, with the required selection of amino acids, the following sequences were synthesized:

- Trp-Phe-Arg-Ala-Leu-His (SEQ ID NO: 1); or
- Phe-Trp-His-Ala-Leu-Arg (SEQ ID NO: 2)

1.2 <u>Cleavage process from the polymeric support of the peptidyl resins obtained</u>

The N-terminal Fmoc group of the peptidyl resins obtained above, was deprotected, washed with DMF ($5\times1$ min), DCM ($4\times1$ min), and dried under vacuum. Afterwards, the peptidyl resins were dispersed in 1% TFA/DCM to obtain the linear peptides with side chain protection groups.

1.3 <u>Cyclization of the amide bond</u>

The cyclization by amide bonds of the peptides was achieved in dilute DMF/DCM peptide solutions ($5\times10^{-4}$ M), with the presence of BOP/DIEA for 15 hours. The cyclization was monitored by HPLC/MS and once it was completed, the solvents were removed by rotary evaporation or lyophilization.

1.4 Deprotection of the cyclic peptide

Weights were normalized. 200 mg of the cyclic peptide obtained in c) were treated with 5 mL of TFA/1,2-ethanedithiol/thioanisole/phenol/$H_2O$/TIS (68.5/10/10/5/3.5/1, v/v) for 4 hours at 30°C under stirring. The peptide was then precipitated in cold ether and dried in vacuo to obtain the final cyclic peptide.

1.5 Characterization of the cyclic peptides

HPLC analysis of the peptides obtained was carried out with a Shimadzu equipment (Kyoto, Japan) using a reverse-phase column (150x4.6 mm, XBridge Peptide BEH C18, 3.5 $\mu$m, Waters, USA) in gradients of MeCN (+0.036% (v/v) TFA) in $H_2O$ (+0.045% (v/v) TFA) at a flow rate of 1.25 mL/min and detection was carried out at 220 nm. All peptides showed a purity exceeding 80%. The identity of the peptides obtained was confirmed by ESI-MS in a Water ZQ 4000 detector using MeOH as the mobile phase and a flow rate of 0.2 mL/min. Results obtained demonstrated that peptides SEQ ID NO: 1 (Peptide A) and SEQ ID NO: 2 (Peptide B), were correctly and effectively synthesized.

## Example 2. Toxicity of the cyclic peptides

Toxicity of cycled peptides A and B was analyzed on human dermal fibroblasts (HDFa) and adult human epidermal keratinocytes HEKa cells by MTT assay. A stock solution of the peptide was prepared in distilled water at 25 mg/mL. This stock solution was further diluted to obtain the final working concentrations (0.001, 0.005, 0.01, 0.05, 0.1 and 0.5 mg/mL) in the corresponding culture medium. Untreated cells were used as negative control samples (Basal).

Briefly, HEKa and HDFa cells were seeded in 96-well plates at a concentration of $1 \times 10^5$ cells/mL in a final volume of 200 $\mu$L of medium (20,000 cells/well). HEKa cells were incubated in Dermal Cell Basal Medium supplemented with the Keratinocytes Growth Kit; and HDFa cells were cultured in Medium 106 supplemented with 2% of LSGS. Cells were incubated at 37°C, 5% $CO_2$ and saturated humidity.

After 24 h of incubation, cells were treated with different concentrations of each peptide (0.001, 0.005, 0.01, 0.05, 0.1 and 0.5 mg/mL) in triplicates, for MTT evaluation. After 24 h of treatment with the peptide, medium was completely removed, and 100 $\mu$L of 0.5 mg/mL MTT solution in supplemented medium were added to each well and cells were incubated at 37°C for 3 h. After incubating with MTT, the medium of each well was aspirated and 150 $\mu$L of DMSO were added in order to solubilise the formazan crystals formed. The plates were placed on a shaker for 5 minutes for complete solubilisation of the crystals and then the absorbance at 570 nm of each well was determined, using the microplate reader Multiskan FC, which is directly proportional to the number of living cells in culture.

Absorbance values were normalized using the data obtained for non-treated cells (control), obtaining the % of viability shown in Tables 1 and 2. Each condition was performed in triplicate (n=3) in 3 independent experiments (N=3).

Concentrations of peptide for which viability is above 80% are considered safe for efficacy testing. On the contrary, concentrations for which viability goes below 80% are considered potentially toxic and are not used for efficacy testing purposes.

**Table 1:** Toxicity of cycled peptides on HDFa cells.

| Concentration peptide | Cell viability | |
|---|---|---|
| | Peptide A | Peptide B |
| Control | 100 % | 100 % |
| 0.001 mg/mL | 101.47 % | 100.42 % |
| 0.005 mg/mL | 102.73 % | 99.77 % |
| 0.01 mg/mL | 94.48 % | 92.93 % |
| 0.05 mg/mL | 103.48 % | 91.90 % |
| 0.1 mg/mL | 92.28 % | 88.18 % |
| 0.5 mg/mL | 81.48 % | 73.74 % |

**Table 2:** Toxicity of cycled peptides on HEKa cells.

| Concentration peptide | Cell viability | |
|---|---|---|
| | Peptide A | Peptide B |
| Control | 100 % | 100 % |

(continued)

| Concentration peptide | Cell viability | |
| --- | --- | --- |
| | Peptide A | Peptide B |
| 0.001 mg/mL | 102.87 % | 101.71 % |
| 0.005 mg/mL | 108.26 % | 102.90 % |
| 0.01 mg/mL | 108.84 % | 106.59 % |
| 0.05 mg/mL | 93.77 % | 91.38 % |
| 0.1 mg/mL | 88.32 % | 85.79 % |
| 0.5 mg/mL | 75.03 % | 61.84% |

From these results it is evidenced that the peptides maximum non-toxic concentration is 0.1 mg/mL.

*Example 3. Analysis of the effect of cycled peptide on the expression of aging-related genes in Human Epidermal Keratinocytes and Human Dermal Fibroblasts*

To determine whether cycled peptides have antiaging effect, the potential of this peptide to modulate the expression of several aging-related genes in human epidermal keratinocytes from adult (HEKa) and human dermal fibroblasts from adult (HDFa) *in vitro* were analyzed.

HEKa cells were incubated in the presence of 0.01 mg/mL cycled peptide B for 24 hours and HDFa cells were treated with 0.05 mg/mL of cycled peptide B for 6 h. Then, cells were lysed, and RNA was extracted (RNeasy mini kit, Qiagen). Purified RNAs were retro-transcribed (High-capacity cDNA reverse transcription kit, Applied Biosystems). RT-qPCR was performed with the panel of TaqMan assay probes (Thermo Fisher Scientific) using StepOne plus Real time PCR instrument. Amplification was performed following Arya M et al. (2005. Basic principles of real-time quantitative PCR. Expert Rev Mol Diagn 5(2): 209-19). The obtained data were analyzed using the ΔΔCt method, which provides the target gene expression values as fold changes in the treated sample compared with an untreated basal sample. Both samples were normalized with the relative expression of the housekeeping gene GAPDH.

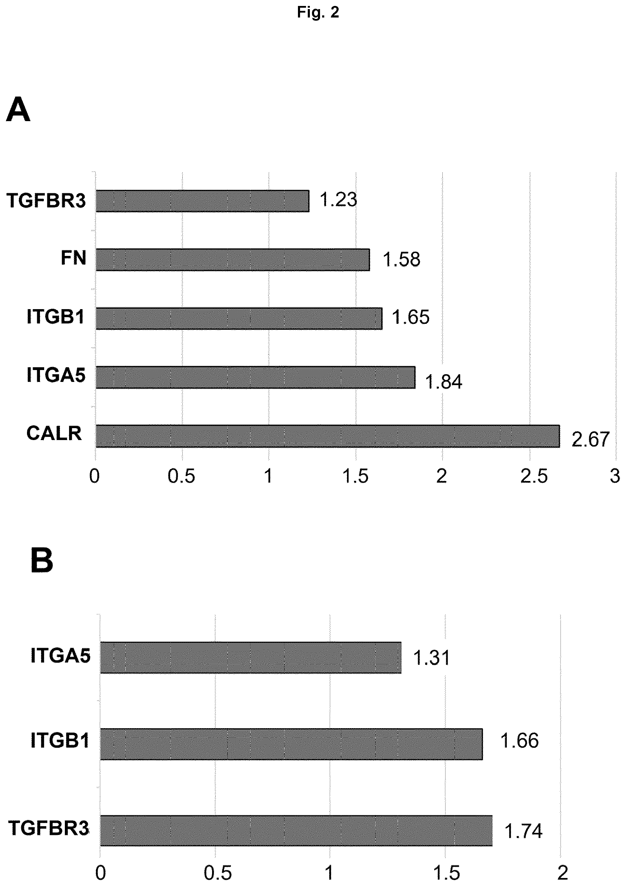

**Figure 2A** shows that HEKa cells treated with the peptide showed upregulation of TGFBR3, FN, ITGB1, ITGA5 and CALR genes. On the other hand, HDFa cells treated with the peptide (**Figure 2B**) showed TGFBR3, ITGB1 and ITGA5 genes upregulated.

**Table 3.** Mean normalized expression and fold change *vs.* Basal for each gene analyzed in HEKa treated with 0.01 mg/mL cycled peptide B for 24 h and HDFa treated with 0.05 mg/mL the same peptide for 6 h.

| | Mean normalized expression | Fold change *vs.* Basal |
|---|---|---|
| TGFBR3 Basal | 2.07E-02 | 1.23 |
| TGFBR3 0.01 mg/mL peptide B | 2.54E-02 | |
| FN Basal | 2.08E-01 | 1.58 |
| FN 0.01 mg/mL peptide B | 3.28E-01 | |
| ITGB1 Basal | 4.34E-01 | 1.65 |
| ITGB1 0.01 mg/mL peptide B | 7.16E-01 | |
| ITGA5 Basal | 6.22E-02 | 1.84 |
| ITGA5 0.01 mg/mL peptide B | 1.15E-01 | |
| CALR Basal | 2.55E-01 | 2.67 |
| CALR 0.01 mg/mL peptide B | 6.83E-01 | |
| ITGA5 Basal | 1.96E-01 | 1.31 |
| ITGA5 0.05 mg/mL peptide B | 2.57E-01 | |
| ITGB1 Basal | 6.12E-01 | 1.66 |
| ITGB1 0.05 mg/mL peptide B | 1.02E+00 | |
| TGFBR3 Basal | 4.60E-03 | 1.74 |
| TGFBR3 0.05 mg/mL peptide B | 8.00E-03 | |

The peptide improved the expression of several aging-related genes. It is remarkable the increase of CALR expression. All these results strongly suggest that this peptide can be used for the development of cosmetic products with antiaging effect.

***Example 4. Analysis of the effect of cycled peptide on calreticulin expression in Human Epidermal Keratinocytes***

The effect of the cycled peptide on the expression of calreticulin (CALR) in human epidermal keratinocytes from adult (HEKa) was analyzed through fluorescence microscopy evaluation.

HEKa cells were cultured on coverslips in Dermal Cell Basal Medium (DCBM) at a density of 50.000 cell/well and incubated at 37 °C in a humidified atmosphere with 5% $CO_2$. When cell confluence was around 80%, cells were treated with cycled peptide B at 0.005 mg/mL for 24 h. After treatment, samples were washed twice with PBS, fixed with 4% formaldehyde and permeabilized with Triton X-100 0.2%. Next, samples were blocked for 1 hour with PBS/BSA 5%, in order to avoid nonspecific antibody binding.

For CALR staining, samples were incubated for 90 min with Calreticulin Polyclonal antibody diluted 1:500 in PBS/BSA 1%, at room temperature. After proper washing with PBS, cells were incubated with 5 $\mu$g/mL of the secondary antibody Goat anti-Rabbit IgG Alexa Fluor 488 (recognition of primary antibody), during 1 hour at room temperature and dark conditions. In some samples, 0.4X Phalloidin-TRITC was added in order to stain actin filaments.

Finally, samples were washed three times with PBS and coverslips were mounted using Prolong-Gold with DAPI, which strongly binds DNA (nuclei staining), and samples were kept protected from light at 4°C until microscopic images were acquired. Microscopic images were acquired using 40x objective. Immunofluorescence was performed on three replicates for each condition and images of at least five different fields from each coverslip were acquired using the same settings.

Images were analyzed using Image J software. Shortly, threshold was adjusted to select CALR staining and mean fluorescent was measured. The number of cells was counted using the DAPI staining. CALR mean fluorescent intensity was divided by the number of cells. Finally, all data was normalized as follows to obtain the % of CALR compared to untreated cells (Basal):

$$\% \ vs \ Basal = \frac{fluorescence \ per \ cell \ in \ treated \ samples}{fluorescence \ per \ cell \ in \ untreated \ samples} \ x \ 100$$

Statistical data analysis was performed using Student t-test by comparison of % fluorescence from peptide B treated samples vs. untreated samples, (*, $p < 0.05$; **, $p < 0.01$ and ***, $p < 0.001$).

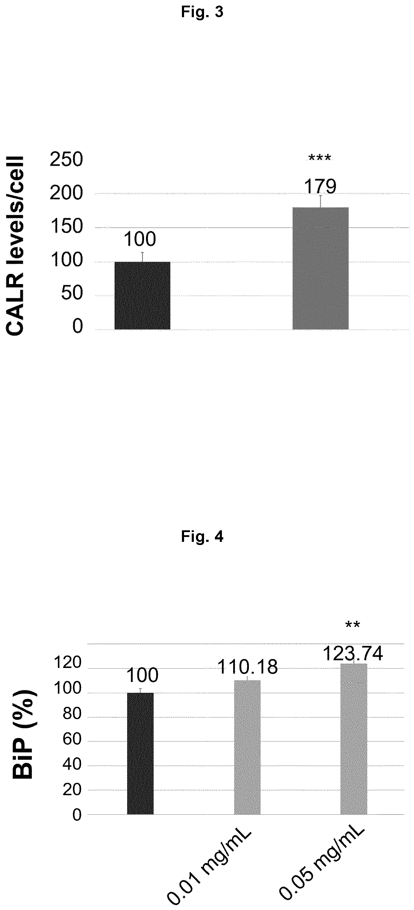

**Figure 3** shows the fluorescence quantification of images of CALR fluorescence on Human Epidermal Keratinocytes after 24 hours of treatment with 0.005 mg/mL of cycled peptide B. Representative images were acquired with a fluorescence microscope. Graphic shows the mean $\pm$ standard error of the mean (SEM).

The treatment of HEKa cells with the cycled peptide increased CALR levels up to 79% when compared with untreated cells. Therefore, treatment of HEKa cells with the cycled peptide results into a significant increase of CALR expression and these results suggest a potential role of the peptide as an enhancer of proper protein folding and UPR, which can be used for antiaging products development.

**Table 4.** Data of the analysis of the results shown in Figure 3B for HEKa cells.

|  | % Mean | ± SEM | p-value (two-tailed) | p-value summary |
|---|---|---|---|---|
| **Basal** | 100.00 | 13.85 | - | - |
| **0.005 mg/mL peptide B** | 179.45 | 17.39 | 0.0010 | *** |

In conclusion, treatment of HEKa cells with 0.005 mg/mL of the cycled peptide results into a significant increase of CALR levels. These results suggest a potential role of the peptide to modulate proper protein folding and UPR, which is of great interest for antiaging products development.

Additionally, expression of CALR after treatment with 0.01 mg/mL of cycled peptides A and B for 24 hours in HEKa cells was analyze by qPCR as described in example 3.

**Table 5.** CALR expression after treatment with peptides A and B

|  | Ratio | Effect |
|---|---|---|
| **CALR Basal** | 1.00 | No effect |
| **Linear peptide B** | 1.06 | No effect |
| **Cyclic peptide B** | 1.78 | Upregulation |
| **Cyclic peptide A** | 1.34 | Upregulation |

***Example 5: Analysis of the effect of cycled peptides on BiP production in human epidermal keratinocytes***

The effect of cycled peptides was determined on the production of human immunoglobulin binding protein (BiP) in human epidermal keratinocytes from adult (HEKa), through quantification of this protein *in vitro* in cell culture lysates by enzyme linked immunosorbent assay (ELISA).

The endoplasmic reticulum (ER) represents the entry point into the secretory pathway where nascent proteins encounter a specialized environment for their folding and maturation. In order to control these processes, the unfolded protein response (UPR) exists, as mentioned previously. It is a signaling system that detects misfolded proteins within the endoplasmic reticulum (ER) and coordinates a cellular response that aims to restore protein homeostasis (Kopp MC et al. (2019). UPR proteins IRE1 and PERK switch BiP from chaperone to ER stress sensor. Nat Struct Mol Biol 26(11): 1053-62; Pobre KFR et al. (2019). The endoplasmic reticulum (ER) chaperone BiP is a master regulator of ER functions: Getting by with a little help from ERdj friends. J Biol Chem 294(6):2098-108).

During aging, there is a decline in the ability of the cell to handle protein folding, accumulation and aggregation, and the function of UPR is compromised. It is caused by a progressive failure of the chaperoning systems and a decline in many of its components, so that the UPR activation cannot rescue the ER stress (Estébanez B et al. (2018). Endoplasmic Reticulum Unfolded Protein Response, Aging and Exercise: An Update. Front Physiol. 9:1744). Therefore, improving UPR and folding capacity can be an interesting antiaging strategy.

In this study the BiP levels in human epidermal keratinocytes treated with cycled peptide B was evaluated, through quantification of this protein in vitro in cell culture lysates by enzyme linked immunosorbent assay (ELISA).

HEKa cells were seeded in a 24-well plate at a density of 50.000 cells/well and maintained at standard culture conditions (Supplemented Dermal Cell Basal Medium, DCBM; 37°C, 95% RH, 5% $CO_2$) for 24 hours. Then, keratinocytes were treated for 24 hours with or without the presence of 0.01 or 0.05 mg/mL of cycled peptide A or peptide B. After treatment, cells were lysed with RIPA buffer and lysates were collected and centrifuged in order to eliminate cell debris and were kept at -80°C until they were used for BiP quantification with BiP ELISA Kit according to manufacturer instructions. In order to normalize data, total protein content of each sample was quantified through Micro BCA Protein Assay kit according to manufacturer instructions.

Each condition was performed at least in triplicate (n=3) in three independent experiments (N=3). For all data, mean values of the different experiments and SEM values are shown. All data was first normalized as follows:

$$BiP\ levels/cell = \frac{BiP\ levels\ per\ well}{Total\ protein\ (BCA)\ per\ well}$$

$$\%\ vs\ Basal = \frac{BiP\ per\ cell\ in\ treated\ samples}{BiP\ per\ cell\ in\ untreated\ samples}\ x\ 100$$

Statistical data analysis was performed using Student t-test by comparison of % BiP expression from cells untreated (Basal) vs. treated with cycled peptide samples, *p<0.05, **p<0.01, ***p<0.001.

**Figure 4** and Table 6 shows that the treatment of HEKa cells with the cycled peptide showed an increase of BiP levels in a concentration dependent manner, reaching up to 24% increment when compared with untreated cells (Basal). Graphic shows the mean $\pm$ standard error of the mean (SEM).

**Table 6.** Data of the analysis of results shown in Figure 4. Data represent the mean of three independent experiments.

|  | % Mean | ± SEM | p-value (two-tailed) | p-value summary |
|---|---|---|---|---|
| **Basal** | 100.00 | 3.71 | - | - |
| **0.01 mg/mL peptide B** | 110.18 | 4.20 | 0.0776 | - |
| **0.05 mg/mL peptide B** | 123.74 | 7.04 | 0.0029 | ** |

Treatment of HEKa cells with the cycled peptide results into a statistically significant increase of BiP production. These results suggest a potential role of these peptides to improve the cellular response to endoplasmic reticulum stress, with possible applications for antiaging products development.

***Example 6: Analysis of the effect of cycled peptides on caspase 3 activity under endoplasmic reticulum stress in human epidermal keratinocytes***

Caspase 3 (CASP3) is a protein member of the cysteine-aspartic acid protease family. Sequential activation of caspases plays a central role in the execution-phase of cell apoptosis. It was evaluated the effect of the cycled peptide B on the caspase 3 activity in human epidermal keratinocytes from adult (HEKa) subjected to endoplasmic reticulum (ER) stress.

Brefeldin (BFA) is a well-known ER-stress inducer, which causes caspase 3 activation, leading to apoptosis (Sandow JJ et al. (2014). ER stress does not cause upregulation and activation of caspase-2 to initiate apoptosis. Cell Death Differ. 21 (3):475-80).

HEKa cells were seeded in a black 96-well plate at a density of 18.000 cell/well in Dermal Cell Basal Medium (DCBM) and incubated at 37 °C in a humidified atmosphere with 5% $CO_2$. After 24 h of incubation, cells were treated with 0.15 $\mu$g/mL BFA alone or in the presence of 0.005 mg/mL of the cycled peptide B and returned to the incubator for 24 h more. Untreated cells (Basal) were used as negative control samples. 24 hours after treatment, caspase 3 activity was evaluated using Caspase-Glo® 3/7 Assay System, following manufacturer instructions.

Each condition was performed at least in triplicates (n=3) in three independent experiments (N=3). For all data, mean values of relative fluorescence units (RFU) of the different experiments and SEM values are shown. All data was normalized as follows:

$$\%\ vs\ BFA = \frac{RFU\ treated\ cells}{RFU\ BFA\ treated\ cells}\ x\ 100$$

Statistical data analysis was performed using Student t-test by comparison of % activity from test substance vs BFA-treated cells, *$p<0.05$, **$p<0.01$, ***$p<0.001$.

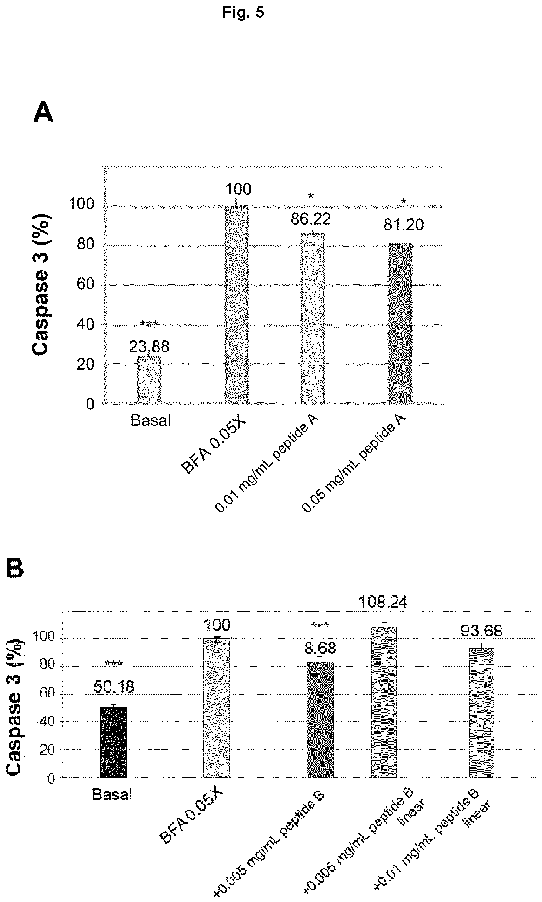

**Figure 5** shows the caspase 3 activity in HEKa cells after treatment with cycled peptide (A), the linear peptide B and the cycled peptide B (B). As may be observed, the cycled peptides A and B significantly reduce caspase 3 activity, protecting cells from apoptosis caused by ER stress. This result suggests an anti-ageing effect of the peptide. The reduction of the caspase expression is not only significantly decreased over the BFA-treated cells, but also over the effect of the linear peptide, showing an unexpected effect of the cycled peptide improved over its linear counterpart.

### *Example 7. Gene expression of aging related genes*

HDFa cells were seeded in duplicate (n=2) in 12-well plates at a concentration of 200.000 cells/well in 106 Medium supplemented with 2% LSGS. Then, cells were incubated at 37 °C in a humidified atmosphere with 5% $CO_2$. Next, cells were treated with cycled peptide B at different non-toxic concentrations for 6 or 24 hours. Untreated cells were used as basal control. Cells were then lysed, and replicates were pooled together for RNA extraction using Qiagen RNeasy Mini kit following manufacturer's instructions. Purified RNAs was then quantified by nanodrop, adjusted in concentration and processed for retrotranscription to cDNA using a commercially available kit (High-Capacity cDNA Reverse Transcription kit - Thermofisher Scientific, USA). Resulting cDNA was used to perform a RTqPCR using Taqman technology, and a panel of probes designed to target different type of collagens and their respective matrix metalloproteinases.

Collagen is an essential scaffold protein in the extracellular matrix. It is produced by cells called fibroblasts and it is determinant for maintaining firmness and elasticity to the skin. However, skin collagen content decreases with age and in response to sun exposure due to the increase of collagen degrading enzymes. This decrease in collagen content is one of the characteristic hallmarks associated with the appearance of fine lines and deeper wrinkles.

### 7.1 Expression of collagen genes

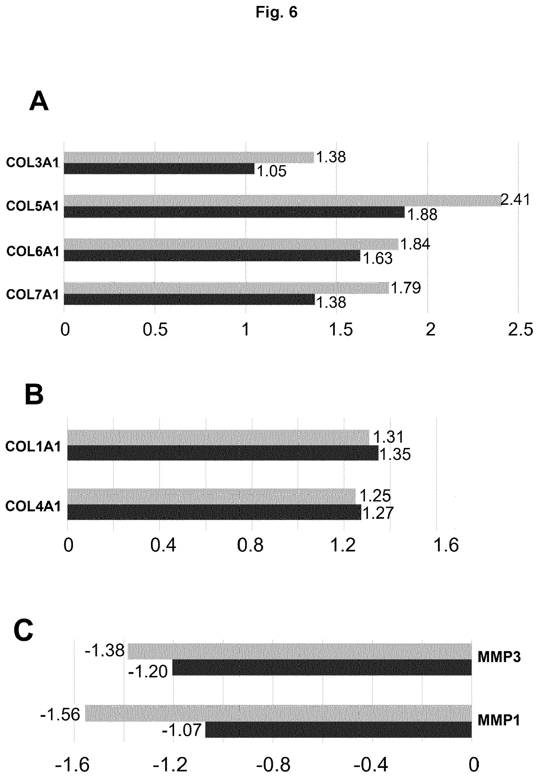

The expression of collagen genes in HDFa cells was measured by qPCR. **Figure 6A** shows the expression of genes COL3A1, COL5A1, COL6A1 and COL7A1 after 6 hours of treatment with 0.001 mg/mL of peptide B (grey) and its linear form (black). **Figure 6B** shows the expression of COL1A1 and COL4A1 after 6 hours of treatment with 0.1 mg/mL of peptide B and its linear form. The results show that the cycled form of the peptide improves the induction of collagen-related genes.

### 7.2 Expression of metalloproteases

Metalloproteases (MMP) are enzymes responsible of extracellular matrix degradation, also important in skin aging. The expression of MMP genes in HDFa cells was measured by qPCR after 24 hours of treatment with 0.01 mg/mL of peptide B and its linear form. **Figure 6C** shows the regulation of MMP3 and MMP1 expression. The results show that the cycled form of the peptide reduces the expression of enzymes involved in collagen degradation.

### *Example 8. Effect of cycled peptides on calreticulin-induced TGF$\beta$3 expression and collagen I synthesis*

For TGF$\beta$3 gene expression assessment, HEKa cells were seeded in 24-well plate at a density of 1000.000 cells/well and incubated at 37 °C in a humidified atmosphere with 5% $CO_2$. Next day, cell culture media was replaced by fresh media containing 0.0025 or 0.01 mg/mL of cycled peptide B. In parallel, HDF cells were seeded on culture plates with or without coverslips (for immunofluorescence or qPCR, respectively) and incubated for 24 h.

Conditioned media from HEKa cells after 48 hours of treatment was then transferred to HDF culture plates. Cells were maintained in culture for additional 24h (for qPCR) or for 4 days (for collagen I immunostaining).

For qPCR, cells were lysed, RNA extracted, and gene expression for TGF$\beta$3 target was obtained as indicated in the previous examples.

For the evaluation of collagen I (COLI) synthesis by immunostaining, HDF cells treated with HEKa conditioned media (as explained above) were washed twice with PBS, fixed with 4% formaldehyde and permeabilized with Triton X-100 0.2%. Next, samples were blocked for 1 hour with PBS/BSA 5%, in order to avoid nonspecific antibody binding. For collagen I staining, samples were incubated for 2 hours with Monoclonal Anti-Collagen, Type I antibody diluted 1:100 in PBS/BSA 1%, at room temperature. After proper washing with PBS, cells were incubated with 5 $\mu$g/mL of the Goat anti mouse IgG (H+L) highly cross-adsorbed secondary antibody, AF488 (recognition of primary antibody), during 2 hours at room temperature and dark conditions. Finally, samples were washed three times with PBS and coverslips were mounted using Prolong-Gold with DAPI, which strongly binds DNA (nuclei staining), and samples were kept protected from light at 4°C until microscopic images were acquired. Microscopic images were acquired using 20x objective.

Immunofluorescence was performed on three replicates for each condition and images of at least five different fields from each coverslip were acquired using the same settings. Images were analyzed using Image J software as mentioned above.

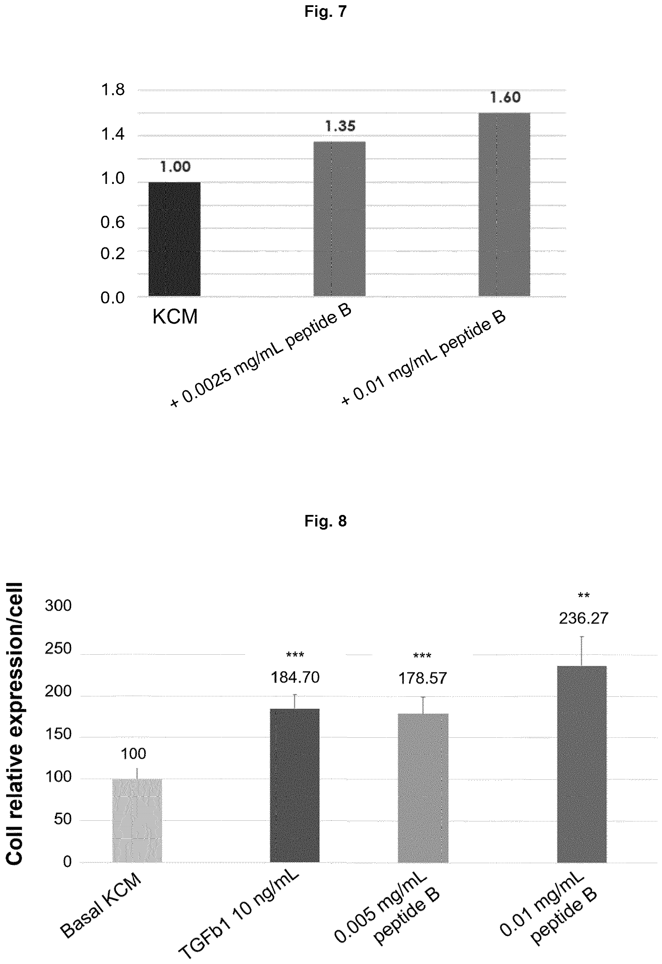

**Figure 7** shows that the expression of TGFβ3 is increased 1.35-fold with 0.0025 mg/mL peptide B over the basal expression in keratinocyte conditioned media (KCM) and 1.60 with 0.01 mg/mL of Peptide B. This indicates that the cycled peptide upregulates the expression of TGFβ3.

**Figure 8** shows that Collagen I (COL1) levels in HDF were significantly increased after 4 days of treatment with conditioned medium from cyclic peptide-treated keratinocytes. The increase in COLI shows that this peptide may be use in skin cosmetic applications.

**Claims**

1. Cycled peptide of between 5 and 10 amino acids capable of increase ER chaperones expression; its acceptable isomers, salts, solvates, derivatives and/or mixtures thereof.

2. The cycled peptide of claim 1, wherein the ER chaperone is CALR.

3. The cycled peptide in accordance with claim 1, wherein the peptide has the sequence of formula (I):
AA1 -AA2-AA3-AA4-AA5-AA6
wherein

   AA1 is selected from an aromatic amino acid;
   AA2 is selected from an aromatic amino acid;
   AA3 is selected from a positively charged amino acid;
   AA4 is Ala;
   AA5 is Leu; and
   AA6 is selected from a positively charged amino acid.

4. The cycled peptide in accordance with claim 3, wherein the peptide has the sequence of formula (I):
AA1-AA2-AA3-AA4-AA5-AA6
wherein

   AA1 is selected from Trp or Phe;
   AA2 is selected from Trp or Phe;
   AA3 is selected from His or Arg;
   AA4 is Ala;
   AA5 is Leu; and
   AA6 is selected from His or Arg.

5. The peptide of claim 4, wherein the peptide is selected from the group consisting of:

   cyclo-Trp-Phe-Arg-Ala-Leu-His (SEQ ID NO:1) and
   cyclo-Phe-Trp-His-Ala-Leu-Arg (SEQ ID NO:2).

6. The peptide of claim 5, wherein the peptide is cyclo- Phe-Trp-His-Ala-Leu-Arg (SEQ ID NO:2).

7. The peptide of any claim of 1 to 6, wherein the peptide is cycled by head-to-tail between amino acids AA1 and AA6.

8. Cosmetic composition comprising a cosmetically effective amount of the cycled peptide of any of claims 1 to 7.

9. The composition of claim 8, wherein the cosmetically effective amount is from 0.0001 % (w/v) to 0.05 % (w/v) of the peptide.

10. The composition of claims 8 or 9, wherein the composition is applied topically in the skin of a subject.

11. Use of the peptide of claims 1 to 7 or the cosmetic composition of claims 8 to 10 to reduce, prevent and/or eliminate signs of skin aging.

12. Use of the peptide of claims 1 to 7 or the cosmetic composition of claims 8 to 10 for the manufacture of a topical cosmetic composition.

13. Pharmaceutical composition comprising the cycled peptide of any of claims 1 to 7.

14. The peptide of any of claims 1 to 7 or the pharmaceutical composition of claim 13 for use as a medicament.

15. The peptide of any of claims 1 to 7 or the pharmaceutical composition of claim 13 for use in a method to reduce, prevent and/or eliminate signs of skin aging in a subject.

Fig. 1

Fig. 2

Fig. 3

Fig. 4

Fig. 5

Fig. 6

## Fig. 7

## Fig. 8

Europäisches
Patentamt

European
Patent Office

Office européen
des brevets

# EUROPEAN SEARCH REPORT

Application Number

EP 23 38 2140

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 114 634 554 A (ZHEJIANG RIVER BAYESIAN PEPTIDE BIO SHARE LTD COMPANY) 17 June 2022 (2022-06-17) * page 1 – page 25; claims; figures; examples; compounds I-1 * | 1-15 | INV. A61K8/64 A61Q19/08 |
| X | CN 114 917 139 A (PROYA COSMETICS CO LTD; ZHEJIANG BAITAI BIOLOGY CO LTD) 19 August 2022 (2022-08-19) * pages 2-21; claims; examples 4, 7, 8, 10, 12, 13, 15; compounds M-type * | 1-15 | |
| A | EP 2 638 059 B1 (ISP INVESTMENTS INC [US]) 11 March 2015 (2015-03-11) * claims; example 2; sequence 5 * | 1-15 | |
| A | WO 2019/149450 A1 (SEDERMA SA [FR]) 8 August 2019 (2019-08-08) * the whole document * | 1-15 | |

TECHNICAL FIELDS SEARCHED (IPC)

A61K
A61Q

The present search report has been drawn up for all claims

| Place of search | Date of completion of the search | Examiner |
|---|---|---|
| Munich | 26 July 2023 | Steffen, Pierre |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 38 2140

This annex lists the patent family members relating to the patent documents cited in the above-mentioned European search report.
The members are as contained in the European Patent Office EDP file on
The European Patent Office is in no way liable for these particulars which are merely given for the purpose of information.

26-07-2023

| Patent document cited in search report | | Publication date | Patent family member(s) | | Publication date |
|---|---|---|---|---|---|
| CN 114634554 | A | 17-06-2022 | NONE | | |
| CN 114917139 | A | 19-08-2022 | NONE | | |
| EP 2638059 | B1 | 11-03-2015 | CN | 103201282 A | 10-07-2013 |
| | | | EP | 2638059 A1 | 18-09-2013 |
| | | | ES | 2536119 T3 | 20-05-2015 |
| | | | FR | 2967160 A1 | 11-05-2012 |
| | | | HK | 1187063 A1 | 28-03-2014 |
| | | | JP | 6179771 B2 | 16-08-2017 |
| | | | JP | 2013542950 A | 28-11-2013 |
| | | | US | 2013288978 A1 | 31-10-2013 |
| | | | WO | 2012062977 A1 | 18-05-2012 |
| WO 2019149450 | A1 | 08-08-2019 | CA | 3087808 A1 | 08-08-2019 |
| | | | CN | 111670027 A | 15-09-2020 |
| | | | EP | 3746041 A1 | 09-12-2020 |
| | | | FR | 3077202 A1 | 02-08-2019 |
| | | | JP | 7285848 B2 | 02-06-2023 |
| | | | JP | 2021513520 A | 27-05-2021 |
| | | | KR | 20200115554 A | 07-10-2020 |
| | | | US | 2021052480 A1 | 25-02-2021 |
| | | | WO | 2019149450 A1 | 08-08-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GANCEVICIENE R et al.** Skin Anti-Aging Strategies. *Dermato-Endocrinology,* 2012, vol. 4, 308-319
- **BAUMANN, L.** Skin ageing and its treatment. *J. Pathol,* 2007, vol. 211, 241-251
- **BRADLEY EJ et al.** Over-the-counter anti-ageing topical agents and their ability to protect and repair photoaged skin. *Maturitas,* 2015, vol. 80, 265-272
- **ZHANG L ; FALLA TJ.** Cosmeceuticals and peptides. *Clinics in dermatology,* 2009, vol. 27, 485-494
- **HETZ C.** The unfolded protein response: controlling cell fate decisions under ER stress and beyond. *Nat Rev Mol Cell Biol.,* 2012, vol. 13, 89-102
- **TAYLOR RC.** Aging and the UPR(ER). *Brain Res.,* 2016, vol. 1648, 588-93
- **MICHALAK M et al.** Calreticulin, a multi-process calcium-buffering chaperone of the endoplasmic reticulum. *Biochem J,* 2009, vol. 417, 651-33
- **OWUSU BY et al.** The role of the endoplasmic reticulum protein calreticulin in mediating TGF-β-stimulated extracellular matrix production in fibrotic disease. *J Cell Commun Signal.,* 2018, vol. 12, 289-99
- **PANDYA UM et al.** Calreticulin exploits TGF-β for extracellular matrix induction engineering a tissue regenerative process. *FASEB J.,* 2020, vol. 34, 15849-74
- **TAOUJI S et al.** Chapter Seventeen - Oligomerization in Endoplasmic Reticulum Stress Signaling. *Progress in Molecular Biology and Translational Science,* 2013, vol. 117, 465-84
- **WHITE C ; YUDIN A.** Contemporary strategies for peptide macrocyclization. *Nature Chem,* 2011, vol. 3, 509-524
- **CLARO B et al.** Design and applications of cyclic peptides. *Peptide Applications in Biomedicine, Biotechnology and Bioengineering,* 2018, 87-129
- CTFA Cosmetic Ingredient Handbook. 2006
- Unusual amino acids in peptide synthesis. **D. C. ROBERTS ; F. VELLACCIO.** The Peptides. Academic Press, 1983, vol. 5
- *Eur. J. Biochem.,* 1984, vol. 138, 937
- **LLOYD WILLIAMS P. et al.** Chemical Approaches to the Synthesis of Peptides and Proteins. *CRC,* 1997
- **KAISER E. et al.** *Anal. Biochem,* 1970, vol. 34, 595598
- **ARYA M et al.** Basic principles of real-time quantitative PCR. *Expert Rev Mol Diagn,* 2005, vol. 5 (2), 209-19
- **KOPP MC et al.** UPR proteins IRE1 and PERK switch BiP from chaperone to ER stress sensor. *Nat Struct Mol Biol,* 2019, vol. 26 (11), 1053-62
- **POBRE KFR et al.** The endoplasmic reticulum (ER) chaperone BiP is a master regulator of ER functions: Getting by with a little help from ERdj friends. *J Biol Chem,* 2019, vol. 294 (6), 2098-108
- **ESTÉBANEZ B et al.** Endoplasmic Reticulum Unfolded Protein Response, Aging and Exercise: An Update. *Front Physiol.,* 2018, vol. 9, 1744
- **SANDOW JJ et al.** ER stress does not cause upregulation and activation of caspase-2 to initiate apoptosis. *Cell Death Differ.,* 2014, vol. 21 (3), 475-80